# EUROPÄISCHE PATENTSCHRIFT

(11) **EP 0 490 218 B1**
(45) Veröffentlichungstag und Bekanntmachung des Hinweises auf die Patenterteilung: **20.04.1994**
(21) Anmeldenummer: 91120706.6
(22) Anmeldetag: 02.12.1991
(51) Int. Cl.: C07C 213/00, C07C 227/04

(54) **Verfahren zur Herstellung von p-Amino-phenolen**
Process for the preparation of p-aminophenols
Procédé pour la préparation des p-aminophénols

(30) Priorität: 13.12.1990 DE 4039862
(43) Veröffentlichungstag der Anmeldung: 17.06.1992
(73) Patentinhaber: BAYER AG, 51368 Leverkusen (DE)
(72) Erfinder: Klausener, Alexander, Dr., W-5190 Stolberg (DE); Landscheidt, Heinz, Dr., W-4100 Duisburg (DE); Blank, Heinz-Ulrich, Dr., W-5068 Odenthal-Glöbusch (DE)

(56) Entgegenhaltungen:
- EP-A- 0 085 890
- DE-A- 2 118 334
- FR-A- 2 555 575
- US-A- 4 307 249
- US-A- 4 885 389
- CHEMICAL ABSTRACTS, vol. 111, no. 19, 6. November 1989, Columbus, Ohio, US; abstract no. 173752K, M.ENOMOTO ET.AL.: 'Preparation of 4-amino-3-fluorophenol' Seite 688 ;Spalte 2 ;
- CHEMICAL ABSTRACTS, vol. 86, no. 9, 28. Februar 1977, Columbus, Ohio, US; abstract no. 55156M, K.TANAKA ET.AL.: 'Ring-Substituted p-Aminophenols' Seite 411 ;Spalte 2 ;

## Beschreibung

Die vorliegende Erfindung betrifft ein Verfahren zur Herstellung von p-Amino-phenolen durch katalytische Hydrierung der zugrundeliegenden aromatischen Nitroverbindungen in einem Reaktionsmedium aus wäßriger Schwefelsäure im Sinne einer Bamberger-Umlagerung, wobei zusätzlich in Gegenwart eines mit Wasser mischbaren organischen Lösungsmittels (Cosolvens) gearbeitet wird.

p-Amino-phenole sind als Zwischenprodukte für die Herstellung von Farbstoffen, pharmazeutischen Wirkstoffen und Pflanzenschutzmitteln von Bedeutung.

Die Herstellung von p-Amino-phenolen durch Hydrieren von Nitroaromaten mit freier p-Position in wäßrigem saurem Medium unter Verwendung von Hydrierkatalysatoren ist als Bamberger-Reaktion in unterschiedlichen Varianten bekannt (Houben-Weyl, 4. Aufl., Band VI/1c (1976), S. 85 bis 117). Im allgemeinen wird hierbei wäßrige Schwefelsäure als Reaktionsmedium gewählt. Die Reaktionsgeschwindigkeit ist dabei im wesentlichen von der Löslichkeit der als Edukte eingesetzten organischen Nitroverbindungen im Reaktionsmedium bzw. ihrer Benetzbarkeit durch das wäßrige System abhängig. Da diese Löslichkeit bzw. Benetzbarkeit bei den üblicherweise als Edukte verwendeten Nitroaromaten jedoch außerordentlich niedrig ist, stellt diese Frage einen kritischen Punkt dar, der die Anwendbarkeit der Bamberger-Reaktion erschwert und einschränkt und in manchen Fällen völlig verhindert.

Aus den genannten Gründen hat es in der Vergangenheit nicht an Versuchen gefehlt, die Benetzung der organischen Nitroverbindung zu verbessern und damit den Verlauf von Bamberger-Reaktionen positiv zu beeinflussen. So wird beispielsweise in US 3.383.416 der Zusatz von Dodecyl-ethylammoniumchlorid und in US 4.307.249 der Zusatz von Dimethyl-alkylaminoxiden als oberflächenaktive Substanzen beschrieben. Gemäß US 3.535.382 soll der Zusatz von auf Phenol basierenden Polyethern des Typs

R-C₆H₄-O-(-CH₂-CH(OH)-CH₂-O-)ₓ-CH₂-CH(OH)-CH₂OH

von Vorteil sein. In DE-AS 19 04 574 wird ein Verfahren beschrieben, bei dem das als Ausgangsmaterial verwendete Nitrobenzol dem Reaktionsgemisch im Verlaufe der Umsetzung zugepumpt wird, um ein Abfallen der katalytischen Aktivität des Katalysators durch (noch) nicht umgesetztes, nahezu wasserunlösliches Nitrobenzol zu vermeiden.

Nachteilig bei den Verfahrensvarianten gemäß den genannten US-Patenten sind die mit den Hilfsstoffen verbundenen Probleme, da diese Hilfsstoffe nach beendeter Reaktion die Isolierung und Reinigung des gewünschten Reaktionsproduktes erschweren, im allgemeinen nicht wiederverwendet werden können, die Abfallproblematik vergrößern und damit eine Verschlechterung der Wirtschaftlichkeit bedingen. Bei dem in der genannten DE-AS beschriebenen Verfahren ist zusätzlich eine aufwendige Steuerung der Dosiervorrichtung erforderlich. Ein weiterer schwerwiegender Nachteil aller obengenannten Verfahren ist es, daß unter den beschriebenen Bedingungen nur die Umsetzung von Nitrobenzol selbst und wenigen weiteren, noch relativ gut wasserlöslichen Nitroaromaten mit im allgemeinen höchstens einem weiteren Substituenten zu befriedigenden Selektivitäten und Raum-Zeit-Ausbeuten führt, daß sich jedoch höhersubstituierte Nitroaromaten nur schlecht und unvollständig umsetzen lassen.

In JP 51/110 528 (1976) werden Bamberger-Reaktionen unter Zusatz von nicht mit Wasser mischbaren Lösungsmitteln, wie Toluol, Dibutylether, 1,1,1-Trichlor-ethan oder Octanol, beschrieben. Hierdurch wird das Problem der schlechten Benetzbarkeit der organischen Nitroverbindung durch die wäßrig-saure Phase jedoch nicht prinzipiell verbessert.

Eine weitere häufig auftretende Schwierigkeit bei Reaktionen wom Bamberger-Typ ist die Tatsache, daß neben dem angestrebten, gegebenenfalls substituierten p-Aminophenol phenol signifikante Mengen des gegebenenfalls substituierten Aminoaromaten gebildet werden. Das Auftreten dieses Nebenproduktes, das im Einzelfall beachtliche und die wirtschaftliche Nutzung des Verfahrens mindernde Ausmaße annehmen kann, wird allgemein auf eine mit der Bamberger-Reaktion konkurrierende Reduktion zurückgeführt, wie sie üblicherweise bei der Einwirkung von Wasserstoff auf Nitroverbindungen in Gegenwart von Edelmetallkatalysatoren abläuft. Um das Verhältnis des gewünschten Aminophenols zu den nicht gewünschten Aminoaromaten vorteilhaft zu beeinflussen, werden dem Reaktionsgemisch bestimmte Stoffe, wie beispielsweise Dimethylsulfoxid, zugesetzt, die den Katalysator teilweise vergiften und seine katalytische Aktivität moderieren bzw. reduzieren und so zu einer verminderten Bildung von Aminoaromaten führen.

Problematisch bei dieser Vorgehensweise ist die Tatsache, daß ein Zusatzstoff in die Reaktion eingebracht wird, dessen Entfernung im Zuge der Aufarbeitung häufig Probleme bereitet. Ferner sind derart desaktivierte Katalysatoren nicht wieder einsetzbar, was einen zusätzlichen Nachteil bedeutet. Beispielhaft für diese Verfahrensweise ist JP 01/121 254; zitiert nach C.A. 111, 173 752, worin in wäßriger Schwefelsäure unter Zusatz von Schwefelverbindungen (z.B. DMSO) 2-Fluor-nitrobenzol zu 4-Amino-3-fluorphenol hydriert wird. Ein weiteres Beispiel hierzu ist das Verfahren DE-OS 21 18 334 zur Herstellung von p-Aminophenol durch Hydrierung von Nitrobenzol in verdünnter Schwefelsäure in Gegenwart von DMSO. Ein Zweistufenverfahren zur Herstellung von p-Amino-phenolen beschreibt EP 85 890. Die erste Stufe der Hydrierung der aromatischen Nitroverbindung zum Phenylhydroxylamin kann hierbei in wäßrigen niederen Alkoholen unter Ausschluß von Säure, aber in Gegenwart einer Schwefelverbindung, wie DMSO, ausgeführt werden, während die zweite Stufe der Umlagerung des Phenylhydroxylamins in Gegenwart von Säure nach Abtrennung der organischen Losungsmittel in der wäßrigen Phase stattfindet.

US 4 885 389 beschreibt die Herstellung von p-Aminophenol durch katalytische Hydrierung von Nitrobenzol in 10 bis 25%iger wäßriger Schwefelsäure unter Zusatz von organischer Säure in einer Menge von 0,1 bis 1 %, bezogen auf das Gewicht des wäßrigen Mediums.

Wird gemäß DE-OS 34 43 385 (entspricht FR 2 555 575 A1) in wäßrig-alkoholischer Schwefelsäure gearbeitet, so erhält man in einer dort gewünschten Weise den diesem Alkohol entsprechenden Ether des p-Aminophenols an Stelle des freien Phenols.

Es bestand daher die Aufgabe, ein Verfahren zur Herstellung der unten genannten, gegebenenfalls substituierten 4-Amino-phenole zu finden, das einerseits eine gute Benetzung des Ausgangsmaterials im Verlaufe der Bamberger-Reaktion ermöglicht und somit zu wirtschaftlich interessanten Reaktionszeiten und Ausbeuten führt, das die Bildung unerwünschter Nebenprodukte vermeidet oder wenigstens weitgehend unterdrückt und das unter Verzicht auf Katalysatorgifte durchgeführt wird, um so Aufarbeitungsprobleme zu vermeiden und die Wiederverwendbarkeit des Katalysators zu gewährleisten.

Diese Aufgabe wird durch das erfindungsgemäße Verfahren gelöst.

Im Gegensatz zu den obengenannten Verfahrensweisen ist das erfindungsgemäße Verfahren dadurch ausgezeichnet, daß es die Anwendung der Bamberger-Reaktion zur Herstellung der unten genannten, gegebenenfalls substituierten 4-Amino-phenole aus den korrespondierenden Nitroaromaten verbessert und auf den Zusatz von Katalysatorgiften verzichtet, so daß der nach beendeter Reaktion abgetrennte Katalysator erneut im erfindungsgemäßen Verfahren wiederverwendet werden kann.

Das erfindungsgemäße Verfahren ist dadurch gekennzeichnet, daß dem Reaktionsgemisch ein mit Wasser mischbares organisches Lösungsmittel (Cosolvens) zugesetzt wird. Derartige Cosolventien verbessern die Durchmischung von organischer und anorganischer Phase ganz erheblich, üben dadurch einen beschleunigenden Effekt auf den Reaktionsablauf aus, lassen sich nach beendeter Umsetzung leicht zurückgewinnen und gegebenenfalls wiederverwenden.

Schließlich ist es ein weiterer überraschender Vorteil des erfindungsgemäßen Verfahrens, daß beim Zusatz solcher Cosolventien teilweise erhebliche Selektivitäts verbesserungen zugunsten der gewünschten substituierten p-Amino-phenole beobachtet werden.

Es wurde ein Verfahren zur Herstellung von p-Aminophenolen der Formel 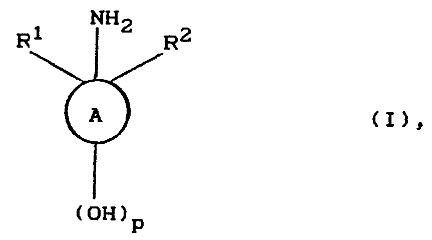
in der
- A: für den Benzol- oder den Naphthalinkern steht,
- p: die p-Stellung zur Aminogruppe anzeigt,
- R¹: Wasserstoff, C₁-C₄-Alkyl, C₁-C₄-Alkoxy, Carboxyl, CO-C₁-C₄-Alkyl oder Halogen bedeutet und
- R²: den Bedeutungsumfang von R¹ mit Ausnahme von Wasserstoff annimmt,

durch katalytische Hydrierung einer aromatischen Nitroverbindung in einem wäßrig-sauren Reaktionsmedium bei erhöhter Temperatur gefunden, das dadurch gekennzeichnet ist, daß als Nitroverbindung eine der Formel 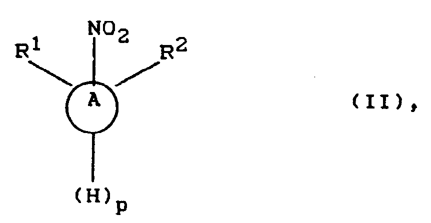
in der
A, p, R¹ und R² die obige Bedeutung haben,

in Gegenwart eines oder mehrerer mit Wasser mischbaren organischen Lösungsmittel(s) aus der Gruppe der C₁-C₄-Alkohole und C₁-C₄-Polyole, der Mono- und Dimethyl- und -ethylether solcher Polyole, Tetrahydrofuran, Dioxan, Aceton, Methylethylketon, N,N-Dimethylacetamid, N,N-Dimethylformamid, N,N-Diethylacetamid und N,N-Diethylformamid umgesetzt wird.

Der positive Effekt, der durch den erfindungsgemäßen Zusatz der genannten mit Wasser mischbaren organischen Lösungsmittel (Cosolventien) auf den Verlauf der Bamberger-Reaktion im Sinne der vermehrten und schnellen Bildung des gewünschten p-Amino-phenols ausgeübt wird, ist durchaus überraschend. Legt man die etwa in DE-OS 34 43 385 beschriebenen Beobachtungen zugrunde, so hätte man beispielsweise in Gegenwart von Alkoholen bevorzugt oder zumindest in erheblichem Maße eine Bildung der entsprechenden p-Alkoxy-aminobenzol-Derivate erwarten müssen.

C₁-C₄-Alkyl ist beispielsweise Methyl, Ethyl, Propyl, Isopropyl, Butyl, Isobutyl, bevorzugt Methyl oder Ethyl, besonders bevorzugt Methyl.

C₁-C₄-Alkoxy ist beispielsweise Methoxy, Ethoxy, Propoxy, Isopropoxy, Butoxy, Isobutoxy, bevorzugt Methoxy oder Ethoxy, besonders bevorzugt Methoxy.

CO-C₁-C₄-Alkyl (Acyl) ist beispielsweise Acetyl, Propionyl, Butyryl, i-Butyryl oder Butylcarbonyl.

Halogen ist beispielsweise Fluor, Chlor oder Brom, bevorzugt Fluor oder Chlor.

Bevorzugte Nitroverbindungen für das erfindungsgemäße Verfahren sind Nitrobenzole der Formel 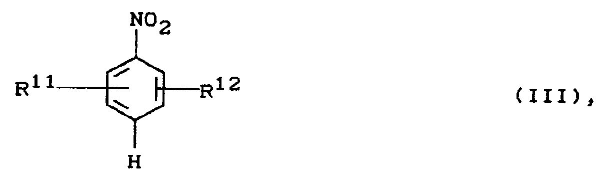
in der
- R¹¹: Wasserstoff, Methyl, Ethyl, Methoxy, Ethoxy, Carboxy, CO-C₁-C₄-Alkyl, Fluor oder Chlor bedeutet und
- R¹²: den Bedeutungsumfang von R¹¹ mit Ausnahme von Wasserstoff annimmt.

Als Cosolventien im Sinne der vorliegenden Erfindung sind beispielsweise niedere Alkohole und Polyole mit 1 bis 4 C-Atomen, wie Methanol, Ethanol, n-und i-Propanol, n-, i-, sec- und tert.-Butanol, Ethylenglykol, 1,2- und 1,3-Propandiol, 1,2-, 1,3-, 1,4-und 2,4-Butandiol sowie 1,2,3-Propantriol geeignet. Weitere Beispiele sind wasserlösliche Ether der genannten Polyole, wie Glykolmono- und -dimethylether, Glykolmono-und -diethylether. Beispiele für weitere erfindungsgemäß einsetzbare Cosolventien sind wasserlösliche cyclische Ether, wie Tetrahydrofuran und Dioxan, mit Wasser mischbare Ketone, wie Aceton und Methylethylketon, wasserlösliche Carbonamide, insbesondere die am Stickstoffatom 2-fach alkylierten, wie N,N-Dimethylacetamid, N,N-Dimethylformamid und die entsprechenden ethylierten niederen Carbonsäureamide. In bevorzugter Weise seien die genannten niederen Alkohole, Ethylenglykol und seine Mono- und Dimethylether der genannten Art und Dioxan genannt. Besonders bevorzugt sei Methanol, Ethanol, Ethylenglykol, Glykolmono- und -dimethylether sowie Dioxan genannt. Die genannten Cosolventien können sowohl einzeln als auch als Gemisch mehrerer von ihnen eingesetzt werden.

Die Menge des organischen, mit Wasser mischbaren Lösungsmittels beträgt das 0,01- bis 3-fache, bevorzugt das 0,03- bis 2-fache, besonders bevorzugt das 0,05- bis 1-fache der Gewichtsmenge der aromatischen Nitroverbindung.

Für das wäßrig-saure Reaktionsmedium kommen starke anorganische oder organische Säuren in Betracht, beispielsweise Schwefelsäure, Phosphorsäure, Salpetersäure, Salzsäure, Perchlorsäure, Methansulfonsäure, Toluolsulfonsäure, Perfluormethansulfonsäure und andere dem Fachmann bekannte. In bevorzugter Weise werden Schwefelsäure oder eine der genannten Sulfonsäuren eingesetzt; in besonders bevorzugter Weise wird Schwefelsäure eingesetzt.

Die Menge der starken Säure beträgt 0,4-10 Äquivalente, bevorzugt 0,5-2 Äquivalente, besonders bevorzugt 0,5-1 Äquivalent, bezogen auf 1 Mol der aromatischen Nitroverbindung.

Die Wassermenge für das wäßrig-saure Reaktionsmedium beträgt das 2- bis 40-fache, bevorzugt das 3- bis 30-fache, besonders bevorzugt das 4- bis 20-fache der Gewichtsmenge der aromatischen Nitroverbindung.

Das erfindungsgemäße Verfahren wird bei einer Temperatur von 50 bis 160°C, bevorzugt 60 bis 140°C, besonders bevorzugt 70 bis 120°C durchgeführt.

Zur katalytischen Hydrierung im Rahmen des erfindungsgemäßen Verfahrens wird unter erhöhtem Druck gearbeitet, wozu in einem Druckreaktor beliebiger Art, wie er dem Fachmann bekannt ist, gearbeitet wird. Selbstverständlich ist ein solcher Reaktor säurefest unter den erfindungsgemäß einzustellenden Reaktionsbedingungen. Als erhöhter Druck sei ein solcher von 2 bar bis zu 50 bar genannt. An diesem erhöhten Druck ist der Wasserstoff-Partialdampfdruck mit 0,1 bis 50 bar, bevorzugt 0,5 bis 30 bar, besonders bevorzugt 1 bis 20 bar beteiligt, kann somit also auch den Gesamtdruck von bis zu 50 bar ausmachen. Die Differenz zwischen dem Wasserstoff-Partialdampfdruck und dem Gesamtdruck ist im allgemeinen der Eigendruck des Reaktionssystems, also der Dampfdruck des Wassers und des zuzusetzenden organischen, mit Wasser mischbaren Lösungsmittels. Hinzu tritt der Dampfdruck der Nitroverbindung. Für den reibungslosen Ablauf einer katalytischen Hydrierung ist weiterhin nach dem Befüllen des Druckreaktors mit den umzusetzenden Stoffen und dem Reaktionsmedium ein Spülen mit Inertgas, wie Stickstoff, Edelgas usw. erforderlich. Auch ein nach Verschließen des Druckreaktors darin verbliebener Rest des inerten Spülgases trägt zum gesamten Druck bei. Im allgemeinen wird hierzu so verfahren, daß der verschlossene Druckreaktor auf die gewünschte Reaktionstemperatur gebracht wird, bevor der Wasserstoff-Partialdampfdruck durch Aufdrücken von Wasserstoff eingestellt wird. Wasserstoff wird dann solange nachgesetzt, wie er durch das Reaktionsgemisch aufgenommen wird.

Als Katalysator für das erfindungsgemäße Verfahren kommen Edelmetalle der Platingruppe, insbesondere Platin und/oder Palladium, in Frage. In gleicher Weise können Verbindungen der Platinmetalle, beispielsweise Platin- und/oder Palladiumverbindungen, eingesetzt werden. Diese Verbindungen werden sodann vom Hydrierwasserstoff zum hydrieraktiven Platinmetall reduziert. Das Platinmetall oder eine Verbindung des Platinmetalls kann mit oder ohne Träger verwendet werden. Träger können beispielsweise Silikagel, Aluminiumoxid, Zeolithe, Molsiebe, Kohle oder andere dem Fachmann bekannte Träger, bevorzugt Kohle, sein. Für den Fall der Mitverwendung eines Trägers beträgt die Metallbelegung 0,05 bis 8 Gew.-%, bevorzugt 0,1 bis 6 Gew.-%, besonders bevorzugt 0,25 bis 5 Gew.-% des Gesamtkatalysators. Der Katalysator mit oder ohne Träger wird in einer solchen Menge eingesetzt, daß 0,001 bis 0,3 Gew.-%, bevorzugt 0,005 bis 0,1 Gew.-%, besonders bevorzugt 0,01 bis 0,1 Gew.-% des Platinmetalls, bezogen auf die umsetzende Nitroverbindung, vorliegen.

Das erfindungsgemäße Verfahren wird mit einem Nitrobenzol oder einem 1-Nitro-naphthalin durchgeführt, das gemäß Formel (II) substituiert sein kann. In bevorzugter Weise wird es mit einem Nitrobenzol durchgeführt, das gemäß Formel (II) substituiert sein kann.

Wichtige aromatische Nitroverbindungen als Ausgangsmaterialien für das erfindungsgemäße Verfahren sind: Nitrobenzol, 2-Nitrotoluol, 3-Nitrotoluol, 2-Chlor-nitrobenzol, 3-Chlor-nitrobenzol, 2-Ethyl-nitrobenzol, 3-Ethyl-nitrobenzol, 2-Acetyl-nitrobenzol, 3-Acetyl-nitrobenzol, 2-Nitrobenzoesäure und Ester, 3-Nitrobenzoesäure und Ester, 2-Fluor-nitrobenzol, 3-Fluor-nitrobenzol, 2,3-Dichlor-nitrobenzol, 2,3-Difluor-nitrobenzol, 2-Chlor-3-fluor-nitrobenzol, 2-Fluor-3-chlor-nitrobenzol, 1-Nitronaphthalin.

Aus solchen Nitroverbindungen entstehen erfindungsgemäß die korrespondierenden p-Aminophenole, bevorzugt die der Benzolreihe.

In bevorzugter Weise seien folgende aromatische Nitroverbindungen der Benzolreihe genannt, aus denen die korrespondierenden p-Aminophenole der Benzolreihe entstehen: 2,3-Dichlor-nitrobenzol, 3-Nitrobenzoesäure, 2-Fluor-nitrobenzol, 3-Fluornitrobenzol, 2-Nitrotoluol, 3-Nitrotoluol, 2-Chlor-nitrobenzol, 3-Chlor-nitrobenzol, 2-Nitrobenzoesäure, 2-Chlor-3-fluor-nitrobenzol, 2-Fluor-3-chlor-nitrobenzol.

In besonders bevorzugter Weise seien genannt:
(i) die erfindungsgemäße Hydrierung von 2,3-Dichlor-nitrobenzol zu 2,3-Dichlor-4-aminophenol und (ii) die erfindungsgemäße Hydrierung von 2-Fluor-nitrobenzol zu 4-Amino-3-fluor-phenol.

2,3-Dichlor-4-aminophenol ist ein wichtiges Zwischenprodukt zur Herstellung von Schädlingsbekämpfungsmitteln (EP 293 718).

4-Amino-3-fluor-phenol ist ein wichtiges Zwischenprodukt zur Herstellung von insektiziden Benzoylharnstoffen des Flufenoxuron-Typs (EP 216 423; EP 277 748). Dieses Aminophenol dient weiterhin der Herstellung verschiedener Phenoxyessigsäureester für Herbizide (JP 01/125 355; zitiert nach C.A. 111, 232 292 k). Weitere Einsatzgebiete dieses Aminophenols sind Herbizide des 3,4,5,6-Tetrahydrophthalimid-Typs (JP 53/073 557; zitiert nach C.A. 90, 6245 p), Haarfärbemittel (GB 1 048 790) und antibakterielle Wirkstoffe (DE-OS 33 38 846).

Die Darstellung von 4-Amino-3-fluor-phenol kann durch Azokupplung diazotierter Sulfanilsäure mit 3-Fluor-phenol und Reduktion der dabei anfallenden 4-(2-Fluor-4-hydroxy-phenylazo)-benzolsulfonsäure mit NaHSO₃ erfolgen (J Chem. Soc. 1964, 473). Auch seine Herstellung durch Bamberger Reduktion von 2-Fluor-nitrobenzol wurde vorgeschlagen (JP 01/121 254; zitiert nach C.A. 111, 173 752), wobei dem Reaktionsgemisch Dimethylsulfoxid zugesetzt wurde.

Die oben genannten Nachteile bei der Anwendung von Verfahren des Standes der Technik treffen auch auf die Herstellung von 2,3-Dichlor-4-aminophenol und 4-Amino-3-fluor-phenol zu.

Das gesamte Reaktionsgemisch wird während der Wasserstoffaufnahme intensiv durchmischt. Hierzu ist der Druckreaktor mit einem Rührer oder mit einer Hubeinrichtung ausgestattet oder als Schüttelautoklav ausgeführt.

Zur Aufarbeitung wird der Katalysator durch Filtration, Abdekantieren oder Zentrifugieren vom restlichen Reaktionsgemisch abgetrennt; das restliche Reaktionsgemisch kann dann in grundsätzlich bekannter Weise, etwa durch Phasentrennung und anschließende Destillation, Kristallisation oder Chromatographie aufgearbeitet werden.

### Beispiele

Die flüssigkeitschromatographischen Analysen (HPLC) wurden als quantitative Bestimmungen unter Vergleich mit eingeeichten Referenzsubstanzen durchgeführt.

### Beispiel 1

43,2 g (0,225 Mol) 2,3-Dichlor-nitrobenzol, 30,0 g (0,31 Mol) Schwefelsäure, 0,5 g Pt (5 %/C), 440 ml Wasser und 10 ml Methanol wurden in einem 1,3 l-Emailleautoklaven auf 115°C erhitzt. Unter intensivem Rühren wurden 10 bar Wasserstoff aufgedrückt. Nach 5,5 Stunden war die Reaktion beendet, und die flüssigkeitschromatographische Analyse des Reaktionsgemisches zeigte folgendes Ergebnis:
79 % 2,3-Dichlor-4-amino-phenol
21 % 2,3-Dichlor-anilin.

### Beispiel 2

28,8 g (0,15 Mol) 2,3-Dichlor-nitrobenzol, 20,0 g (0,21 Mol) Schwefelsäure, 0,5 g Pt (5 %/C), 300 ml Wasser und 50 ml Ethylenglykol wurden in einem 1 l-Glasautoklaven auf 105°C erhitzt. Unter intensivem Rühren wurden 2,6 bar Wasserstoff aufgedrückt. Nach 8 Stunden war die Reaktion beendet, und die flüssigkeitschromatographische Analyse des Reaktionsgemisches zeigte folgendes Ergebnis:
64 % 2,3-Dichlor-4-amino-phenol
36 % 2,3-Dichlor-anilin.

### Beispiel 3

96,0 g (0,5 Mol) 2,3-Dichlor-nitrobenzol, 45,0 g (0,46 Mol) Schwefelsäure, 0,5 g Pt (5 %/C), 400 ml Wasser und 50 ml Methanol wurden in einem 1,3 l-Emailleautoklaven auf 115°C erhitzt. Unter intensivem Rühren wurden 10 bar Wasserstoff aufgedrückt. Nach 7 Stunden war die Reaktion beendet.

Zur Aufarbeitung wurden die Lösungsmittel unter vermindertem Druck abdestilliert. Der verbleibende Rückstand wurde aus 300 ml Wasser unter Zusatz von 2,0 g Aktivkohle umkristallisiert.
Ausbeute: 81 g (59 %) 2,3-Dichlor-4-amino-phenolhydrogensulfat

### Vergleichsbeispiel 1

28,8 g (0,15 Mol) 2,3-Dichlor-nitrobenzol, 350 ml Wasser, 20 g (0,21 Mol) Schwefelsäure und 1 g Pt (5 %/C) wurden in einem 1 l-Glasautoklaven auf 120°C erhitzt. Unter intensivem Rühren wurden 3 bar Wasserstoff aufgedrückt. Die Reaktion war nach 6 Stunden beendet und die flüssigkeitschromatographische Analyse zeigte folgendes Ergebnis:
35 % 2,3-Dichlor-4-amino-phenol
65 % 2,3-Dichlor-anilin.

### Beispiel 4

33,4 g (0,20 Mol) 3-Nitro-benzoesäure, 440 ml Wasser, 30 ml Methanol, 34,0 g (0,347 Mol) Schwefelsäure und 1,5 g Pt (5 %/C) wurden in einem 1,3 l-Emailleautoklaven auf 120°C erhitzt. Unter intensivem Rühren wurden 3 bar Wasserstoff aufgedrückt. Nach 75 Minuten war die Reaktion beendet, und die flüssigkeitschromatographische Analyse zeigte folgendes Ergebnis:
71 % 5-Amino-salicylsäure
29 % 3-Amino-benzoesäure.

### Vergleichsbeispiel 2

33,4 g (0,20 Mol) 3-Nitro-benzoesäure, 470 ml Wasser, 34,0 g (0,347 Mol) Schwefelsäure und 1,5 g Pt (5 %/C) wurden in einem 1,3 l-Emailleautoklaven auf 120°C erhitzt. Unter intensivem Rühren wurden 3 bar Wasserstoff aufgedrückt. Nach 90 Minuten war die Reaktion beendet, und die flüssigkeitschromatographische Analyse zeigte folgendes Ergebnis:
66 % 5-Amino-salicylsäure und
22 % 3-Amino-benzoesäure (Rest nicht identifizierte Stoffe).

### Beispiel 5

Ein Gemisch aus 28,20 g (0,20 Mol) 2-Fluor-nitrobenzol, 450 ml Wasser, 20 ml Ethanol und 27,00 g (0,28 Mol) Schwefelsäure wurde unter Rühren und Zusatz von 1,50 g Pt (5 % auf Norit CNl, Wassergehalt 64,1 %) im geschlossenen Emailleautoklaven auf 120°C erhitzt. Dabei stellte sich ein Eigendruck von ca. 2 bar ein. Man preßte Wasserstoff auf (Partialdruck 3 bar) und hydrierte bis zur Beendigung der H₂-Aufnahme (5,5 Stunden). Nach dem Abkühlen wurde filtriert, der Filterrückstand mit wenig warmem Wasser nachgewaschen, und das Filtrat mit 2 x 100 ml Ethylacetat extrahiert, um Reste nicht umgesetzten Ausgangsmaterials zu entfernen. Die wäßrige Phase wurde durch Zusatz konzentrierter Natronlauge (ca. 38,5 ml) auf pH 9 gebracht und ca. 4 Stunden lang mit Ethylacetat perforiert. Der organische Extrakt wurde eingeengt und der verbleibende Rückstand unter vermindertem Druck getrocknet.
Ausbeute: 21,0 g Rohprodukt
Analytik: HPLC 78,4 % 4-Amino-3-fluor-phenol und 13,9 % 2-Fluor-anilin (Rest nicht identifizierte Stoffe).

Das dunkel gefärbte Rohprodukt wurde ca. 1 Stunde lang mit siedendem Petrolether ausgekocht. Man saugte ab und trocknete den Filterrückstand unter vermindertem Druck.
- Ausbeute:: 15,0 g (60 % der theoretischen Ausbeute)
- Analytik: HPLC: 98,5 % 4-Amino-3-fluor-phenol

### Vergleichsbeispiel 3

Ein Gemisch aus 28,20 g (0,20 Mol) 2-Fluor-nitrobenzol, 470 ml Wasser und 27,00 g (0,28 Mol) Schwefelsäure wurde unter Rühren und Zusatz von 1,50 g Pt (5 %/C) im geschlossenen Emailleautoklaven auf 120°C erhitzt. Dabei stellte sich ein Eigendruck von ca. 2 bar ein. Man preßte Wasserstoff auf (Partialdruck 3 bar) und hydrierte bis zur Beendigung der H₂-Aufnahme. Nach dem Abkühlen wurde das Reaktionsgemisch flüssigkeitschromatographisch untersucht. Es zeigte sich, daß 35 % des eingesetzten 2-Fluor-nitrobenzols nicht umgesetzt worden waren.

### Beispiel 6

Das Beispiel 5 wurde wiederholt, jedoch wurden nur 440 ml Wasser verwendet, und statt des Ethanols wurden 30 ml Methanol dem Reaktionsgemisch zugesetzt.
Ausbeute: 17,9 g (57,0%); HPLC: 100 % 4-Amino-3-fluorphenol.

### Beispiel 7

Das Beispiel 6 wurde wiederholt, wobei der in Beispiel 6 abgetrennte Katalysator eingesetzt wurde.
Ausbeute: 20,2 g (64,3%); HPLC: 100 % 4-Amino-3-fluorphenol.

### Vergleichsbeispiel 4

Ein aus der Nacharbeitung von JP 01/121 254 wiedergewonnener Katalysator wurde erneut eingesetzt und das Beispiel 5 unter ansonsten gleichen Bedingungen wiederholt. Es zeigte sich, daß das Ausgangsmaterial nicht umgesetzt wurde.

## Patentansprüche

1. Verfahren zur Herstellung von p-Amino-phenolen der Formel 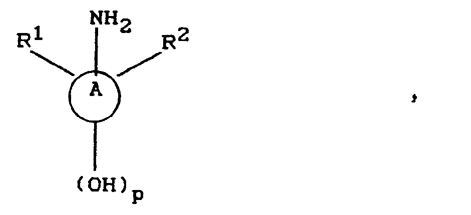 in der
A für den Benzol- oder den Naphthalinkern steht,
p die p-Stellung zur Aminogruppe anzeigt,
R¹ Wasserstoff, C₁-C₄-Alkyl, C₁-C₄-Alkoxy, Carboxyl, CO-C ₁-C₄-Alkyl oder Halogen bedeutet und
R² den Bedeutungsumfang von R¹ mit Ausnhame von Wasserstoff annimmt,
durch katalytische Hydrierung einer aromatischen Nitroverbindung in einem wäßrig-sauren Reaktionsmedium bei erhöhter Temperatur, dadurch gekennzeichnet, daß als Nitroverbindung eine der Formel 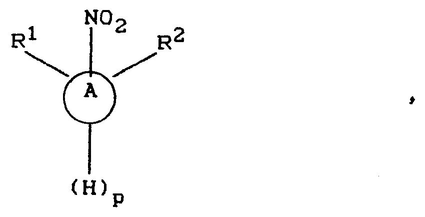 in der
A, p, R¹ und R² die obige Bedeutung haben,
in Gegenwart eines oder mehrerer mit Wasser mischbaren organischen Lösungsmittel(s) aus der Gruppe der C₁-C₄-Alkohole und C₁-C₄-Polyole, der Mono- und Dimethyl- und -ethylether solcher Polyole, Tetrahydrofuran, Dioxan, Aceton, Methylethylketon, N,N-Dimethylacetamid, N,N-Dimethylformamid, N,N-Diethylacetamid und N,N-Diethylformamid umgesetzt wird.

2. Verfahren nach Anspruch 1, dadurch gekennzeichnet, daß als organisches, mit Wasser mischbares Lösungsmittel eines oder mehrere aus der Gruppe der C₁-C₄-Alkohole, des Ethylenglykols und seiner Mono- und Dimethyl- und -ethylether und des Dioxans, bevorzugt aus der Gruppe Methanol, Ethanol, Ethylenglykol, Glykolmono- und -dimethylether und Dioxan, eingesetzt wird.

3. Verfahren nach Anspruch 1, dadurch gekennzeichnet, daß die Menge des organischen, mit Wasser mischbaren Lösungsmittels das 0,01- bis 3-fache, bevorzugt das 0,05- bis 2-fache, besonders bevorzugt das 0,1- bis 1-fache der Gewichtsmenge der aromatischen Nitroverbindung beträgt.

4. Verfahren nach Anspruch 1, dadurch gekennzeichnet, daß Schwefelsäure oder eine organische Sulfonsäure, bevorzugt Schwefelsäure, in einer Menge von 0,4 bis 10 Äquivalenten, bevorzugt 0,5 bis 2 Äquivalenten, besonders bevorzugt 0,5 bis 1 Äquivalent pro Mol der aromatischen Nitroverbindung eingesetzt wird.

5. Verfahren nach Anspruch 1, dadurch gekennzeichnet, daß die Wassermenge das 2- bis 40-fache, bevorzugt das 3- bis 30-fache, besonders bevorzugt das 4- bis 12-fache der Gewichtsmenge der aromatischen Nitroverbindung beträgt.

6. Verfahren nach Anspruch 1, dadurch gekennzeichnet, daß die erhöhte Temperatur 50 bis 160°C, bevorzugt 70 bis 140°C, besonders bevorzugt 90 bis 120°C beträgt.

7. Verfahren nach Anspruch 1, dadurch gekennzeichnet, daß der Wasserstoff-Partialdruck 0,1 bis 50 bar, bevorzugt 0,5 bis 30 bar, besonders bevorzugt 1 bis 20 bar beträgt.

8. Verfahren nach Anspruch 1, dadurch gekennzeichnet, daß als Nitroverbindung Nitrobenzole der Formel 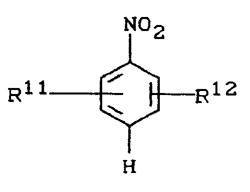 eingesetzt werden, in der
R¹¹ Wasserstoff, Methyl, Ethyl, Methoxy, Ethoxy, Carboxyl, CO-C₁-C₄-Alkyl, Fluor oder Chlor bedeutet und R¹² den Bedeutungsumfang von R¹¹ mit Ausnahme von Wasserstoff annimmt.

## Claims

1. Process for the preparation of p-amino-phenols of the formula 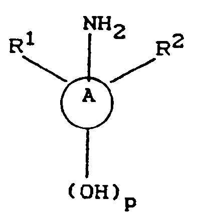 in which
A represents the benzene or naphthalene nucleus,
p indicates the p-position relative to the amino group,
R¹ denotes hydrogen, C₁-C₄-alkyl, C₁-C₄-alkoxy, carboxyl, CO-C₁-C₄-alkyl or halogen and
R² assumes the scope of meaning of R¹ with the exception of hydrogen,
by catalytic hydrogenation of an aromatic nitro compound in an aqueous-acid reaction medium at elevated temperature, characterized in that, as the nitro compound, a compound of the formula 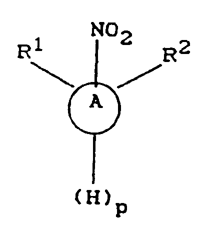 in which
A, p, R¹ and R² have the above meaning,
is reacted in the presence of one or more water-miscible organic solvent(s) from the group comprising C₁-C₄-alcohols and C₁-C₄-polyols, the mono- and dimethyl and mono- and diethyl ethers of such polyols, tetrahydrofuran, dioxane, acetone, methyl ethyl ketone, N,N-dimethylacetamide, N,N-dimethylformamide, N,N-diethylacetamide and N,N-diethylformamide.

2. Process according to Claim 1, characterized in that the organic water-miscible solvent employed is one or more solvents from the group comprising C₁-C₄-alcohols, ethylene glycol and its mono- and dimethyl and mono- and diethyl ethers and dioxane, preferably from the group comprising methanol, ethanol, ethylene glycol, glycol mono- and dimethyl ethers and dioxane.

3. Process according to Claim 1, characterized in that the amount of organic water-miscible solvent is 0.01 to 3 times, preferably 0.05 to 2 times, particularly preferably 0.1 to 1 times, the amount by weight of the aromatic nitro compound.

4. Process according to Claim 1, characterized in that sulphuric acid or an organic sulphonic acid, preferably sulphuric acid, is employed in an amount of 0.4 to 10 equivalents, preferably 0.5 to 2 equivalents, particularly preferably 0.5 to 1 equivalent, per mole of the aromatic nitro compound.

5. Process according to Claim 1, characterized in that the amount of water is 2 to 40 times, preferably 3 to 30 times, particularly preferably 4 to 12 times, the amount by weight of the aromatic nitro compound.

6. Process according to Claim 1, characterized in that the elevated temperature is 50 to 160°C, preferably 70 to 140°C, particularly preferably 90 to 120°C.

7. Process according to Claim 1, characterized in that the hydrogen partial pressure is 0.1 to 50 bar, preferably 0.5 to 30 bar, particularly preferably 1 to 20 bar.

8. Process according to Claim 1, characterized in that nitrobenzenes of the formula 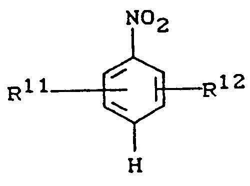 in which
R¹¹ denotes hydrogen, methyl, ethyl, methoxy, ethoxy, carboxyl, CO-C₁-C₄-alkyl, fluorine or chlorine and
R¹² assumes the scope of meaning of R¹¹ with the exception of hydrogen,
are employed as the nitro compound.

## Revendications

1. Procédé pour la préparation de p-aminophénols de formule 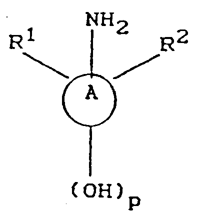 dans laquelle
A représente un noyau benzénique ou un noyau naphtalénique
p indique la position p par rapport au groupe amino,
R¹ représente un atome d'hydrogène, un groupe alkyle en C₁-C₄, un groupe alcoxy en C₁-C₄, un groupe carboxyle, un groupe CO-alkyle en C₁-C₄ ou encore un atome d'halogène,
R² adopte la portée de signification de R¹, à l'exception d'un atome d'hydrogène,
par hydrogénation catalytique d'un composé nitro aromatique dans un milieu réactionnel aqueux-acide à température élevée, caractérisé en ce que, comme composé nitro, on fait réagir un composé répondant à la formule 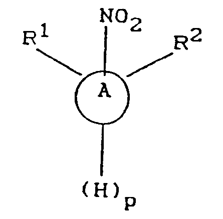 dans laquelle
A, p, R¹ et R² ont la signification indiquée ci-dessus,
en présence d'un ou de plusieurs solvants organiques miscibles à l'eau du groupe comprenant des alcools en C₁-C₄ et des polyols en C₁-C₄, les éthers mono- et di-méthyliques et éthyliques de tels polyols, le tétrahydrofuranne, le dioxanne, l'acétone, la méthyléthylcétone, le N,N-diméthylacétamide, le N,N-diméthylformamide, le N,N-diéthylacétamide et le N,N-diéthylformamide.

2. Procédé selon la revendication 1, caractérisé en ce qu'on met en oeuvre, comme solvant organique miscible à l'eau, un ou plusieurs solvants du groupe comprenant des alcools en C₁-C₄, l'éthylèneglycol et ses éthers mono- et diméthyliques et éthyliques, ainsi que le dioxanne, de préférence du groupe comprenant le méthanol, l'éthanol, l'éthylèneglycol, les éthers mono- et di-méthyliques de glycol, ainsi que le dioxanne.

3. Procédé selon la revendication 1, caractérisé en ce que la quantité du solvant organique miscible à l'eau représente de 0,01 à 3 fois, de préférence de 0,05 à 2 fois, de manière particulièrement préférée de 0,1 à 1 fois la quantité pondérale du composé nitro aromatique.

4. Procédé selon la revendication 1, caractérisé en ce qu'on met en oeuvre l'acide sulfurique ou un acide sulfonique organique, de préférence l'acide sulfurique, en une quantité de 0,4 à 10 équivalents, de préférence de 0,5 à 2 équivalents, de manière particulièrement préférée de 0,5 à 1 équivalent par mole de composé nitro aromatique.

5. Procédé selon la revendication 1, caractérisé en ce que la quantité d'eau représente de 1 à 40 fois, de préférence de 3 à 30 fois, de manière particulièrement préférée de 4 à 12 fois la quantité pondérale du composé nitro aromatique.

6. Procédé selon la revendication 1, caractérisé en ce que la température élevée se situe de 50 à 160°C, de préférence de 70 à 140°C, de manière particulièrement préférée de 90 à 120°C.

7. Procédé selon la revendication 1, caractérisé en ce que la pression partielle d'hydrogène s'élève de 0,1 à 50 bar, de préférence de 0,5 à 30 bar, de manière particulièrement préférée de 1 à 20 bar.

8. Procédé selon la revendication 1, caractérisé en ce que, comme composé nitro, on met en oeuvre des nitrobenzènes de formule 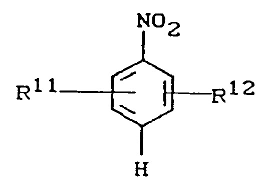 dans laquelle
R¹¹ représente un atome d'hydrogène, un groupe méthyle, un groupe éthyle, un groupe méthoxy, un groupe éthoxy, un groupe carboxyle, un groupe CO-alkyle en C₁-C₄, un atome de fluor ou un atome de chlore, et R¹² adopte la portée de signification de R¹¹, à l'exception d'un atome d'hydrogène.
